# FASCICULE DE BREVET EUROPEEN

(11) **EP 0 451 070 B1**
(45) Date de publication et mention de la délivrance du brevet: **08.11.1995**
(21) Numéro de dépôt: 91430007.4
(22) Date de dépôt: 04.04.1991
(51) Int. Cl.: G01N 33/68, G01N 33/569, G01N 33/74

(54) **Procédé d'identification ou de dosage de protéines et applications**
Verfahren zur Identifizierung oder Bestimmung von Proteinen und Verwendungen dazu
Method for identifying or determining proteins and applications therefor

(30) Priorité: 06.04.1990 FR 9004774
(43) Date de publication de la demande: 09.10.1991
(73) Titulaire: IMMUNOTECH S.A., F-13276 Marseille Cédex 9 (FR)
(72) Inventeur: Jean, Frédéric, F-13006 Marseille (FR); Barbet, Jacques, F-13009 Marseille (FR); Delaage, Michel, F-13001 Marseille (FR)
(74) Mandataire: Rinuy, Santarelli

(56) Documents cités:
- EP-A- 0 135 277
- EP-A- 0 175 360
- EP-A- 0 232 165
- EP-A- 0 345 906
- WO-A-87/07957
- FR-A- 2 383 962

## Description

La présente invention concerne un procédé d'identification ou de dosage de protéines et ses applications.

Le dosage immunologique précis d'une protéine nécessite l'utilisation d'anticorps dont l'affinité permet la détection de quantités généralement faibles d'antigène et dont la spécificité évite les interférences avec les autres constituants du milieu dans lequel on effectue ce dosage. L'obtention de ces anticorps se heurte fréquemment à la difficulté de posséder une source suffisamment abondante et de pureté convenable de l'antigène étudié.

L'utilisation de peptides de synthèse comme immunogènes a permis d'obtenir des anticorps dirigés contre des antigènes rares ou dont on ne connaît que la séquence en acides aminés ou contre des épitopes particuliers, faiblement immunogéniques au sein d'une protéine. Cependant, dans la plupart des cas, les anticorps produits contre un peptide synthétique ne reconnaissent pas la protéine complète avec une affinité suffisante pour établir un dosage immunologique de sensibilité convenable.

De nombreux traitements ont été envisagés pour éliminer les interférences dues aux autres constituants du milieu dans lequel on réalise un dosage immunologique d'une protéine. Une de ces méthodes consiste à effectuer le dosage après un traitement protéolytique de l'échantillon. Cette méthode, telle qu'elle est décrite, permet soit de détruire sélectivement les épitopes présents sur les protéines contaminantes en conservant ceux qui sont portés par la protéine à doser, soit de doser des fragments protéolytiques de la protéine gui portent des épitopes spécifiques, à l'aide d'anticorps obtenus par immunisation avec des fragments purifiés de la protéine clivée (demande de brevet européen N° 0.051.985). Cependant, chacun des deux aspects de cette méthodologie présente des inconvénients. La destruction sélective des épitopes portés par les protéines interférentes ne peut être réalisée que si l'on connaît précisément la localisation des épitopes reconnus par les anticorps du dosage et la nature des protéines contaminantes, ou par une étude extensive des différentes techniques de fragmentation protéolytique. En outre, le dosage de fragments protéolytiques à l'aide d'anticorps obtenus par immunisation avec les fragments purifiés nécessite une quantité importante de la protéine native très pure. De plus, la recherche d'anticorps spécifiques implique la caractérisation des fragments protéolytiques purifiés avant leur utilisation en temps qu'immunogène, ou l'étude approfondie de la spécificité des anticorps obtenus après immunisation avec les différents peptides.

Cupo A., Rougon-Rapuzzi G. et Delaage M. (1981) dans Immunochemical detection of vasopressin precursors : artificial processing and quantification along the Hypothalamo-Hypophisial axis, Eur. J. Biochem., 115, 169-174 ont décrit une méthode de dosage du précurseur de la vasopressine au moyen d'anticorps dirigés contre la vasopressine elle-même, en reconstituant le déterminant antigénique avant le dosage par traitement enzymatique et chimique.

Une approche équivalente a été également utilisée avec succès pour le dosage de la Met-enképhaline, en utilisant des anticorps produits contr un fragment naturel du précurseur. Ce dosage est réalisé après un traitement de l'échantillon par l'acide trichloracétique pour éliminer le fragment naturel, suivi d'un traitement par la trypsine (Cupo A., Pontarotti P.A., Jarry T. et Delaage M.A. (1984) dans "A new immunological approach to the detection and the quantitation of the Met-enkephalin precursors in rat brain", Neuropeptides, 4 : 375-387).

WO-A-87 07957 décrit un procédé de dosage par compétition d'une protéine à l'aide d'un anticorps dirigé contre cette protéine, et d'une protéine de synthèse utilisée comme traceur, également reconnue par cet anticorps. Toutefois, ce procédé n'indique pas que la protéine de synthèse puisse être utilisée pour produire des anticorps , et de plus la mise en oeuvre de ce procédé nécessite la synthèse d'une protéine dont la structure est très proche de la protéine naturelle contre laquelle est dirigé l'anticorps, afin que la réactivité de cet anticorps soit équivalente pour la protéine de synthèse et la protéine naturelle. Ce procédé ne permet pas de choisir la protéine de synthèse en fonction d'un traitement enzymatique de l'échantillon susceptible de générer un peptide de structure identique à la protéine de synthèse.

EP-A-0 175 360 décrit un procédé de dosage d'une protéine à l'aide d'un anticorps produit contre un peptide de synthèse dont l'épitope correspondant sur la protéine naturelle est exposé au cours du dosage par l'emploi d'un agent faiblement dénaturant pour la protéine, qui préserve néanmoins la capacité de liaison de l'anticorps.

Ce procédé n'indique pas que le traitement de l'échantillon pourrait être une protéolyse enzymatique qui ne peut être considérée comme une dénaturation partielle de la protéine.

Il est impossible de choisir à l'avance une séquence en acide aminés correspondant à un épitope qui aurait une chance d'être exposé par ce traitement.

Plus simplement, dans la présente invention, l'étude de la structure primaire de la protéine à doser permet le dosage direct et sans modification ultérieure ou traitement préalable, de fragments protéolytiques spécifiques. Elle se distingue des travaux cités par trois différences essentielles : le peptide immunisant est un fragment artificiel, il n'y a pas d'extraction à réaliser pour éliminer un fragment naturel et il n'y a pas de modification chimique à opérer sur les échantillons.

C'est pourquoi la présente demande a pour objet un procédé d'identification ou de dosage de protéines dans lequel l'on prépare des anticorps à l'aide d'un peptide rendu si nécessaire immunogène, puis utilise les anticorps ainsi obtenus pour doser les protéines désirées à partir d'un échantillon les renfermant traité de manière à libérer ledit peptide, caractérisé en ce que ledit peptide est sélectionné à partir d'une séquence connue de ladite protéine en tant que fragment ou partie de fragment de protéine libérable par traitement enzymatique puis est préparé par synthèse.

Un tel procédé possède les avantages liés à l'utilisation d'immunogènes peptidiques de synthèse : facilité de préparation, choix de l'épitope intéressant, et permet de plus d'éviter les problèmes de perte d'affinité rencontrés dans le cas d'un dosage immunologique d'une protéine native car, dans ce cas, l'immunogène correspond parfaitement à l'antigène à doser, puisque par exemple il peut être strictement identique à l'antigène à doser. Par ailleurs, il existe un large éventail de techniques de clivage protéolytique enzymatique, dont les sites de coupure sont spécifiques et facilement identifiables sur la structure primaire de la protéine, permettant un large choix dans la sélection d'un fragment peptidique.

La préparation d'anticorps à l'aide de peptides est bien connue de l'état de la technique ; dans le cas ou lesdits peptides ne sont pas ou sont insuffisamment immunogènes, il est possible de leur conférer ces propriétés, par exemple en fixant lesdits peptides sur une macromolécule, notamment d'origine protéique.

La sélection desdits peptides pourra se faire à partir d'une séquence connue en acides aminés ou déduite de la séquence en acides nucléiques d'un gène correspondant.

Ces peptides correspondront à un fragment d'une protéine telle qu'une hormone ou une protéine virale.

Les peptides seront sélectionnés parce qu'ils correspondent à des fragments protéolytiques qu'il sera possible d'obtenir par un traitement enzymatique tels que des fragments trypsiques obtenus après traitement de ladite protéine par la trypsine.

Par "partie de fragment", l'on entend une partie C-terminale, N-terminale ou médiane du fragment protéique.

Dans ce qui suit, par "fragment", l'on entend fragment ou partie de fragment.

Il est bien sûr possible d'utiliser d'autres traitements enzymatiques simples ou associant plusieurs enzymes, telles que par exemple trypsine, chymotrypsine, proline endopeptidase.

Ces peptides seront également sélectionnés en fonction de leur taille et de leur représentativité par rapport à une protéine unique ou par rapport à un groupe de protéines.

En vue de la mise en oeuvre du procédé selon la présente invention, on peut utiliser un ou plusieurs peptides, par exemple 2, 3 ou 4.

Dans des conditions préférentielles de mise en oeuvre du procédé ci-dessus décrit, le peptide sélectionné à partir d'une séquence connue de protéines en tant que fragment de protéine libérable par un traitement approprié comportera de 6 à 30 acides aminés et de préférence de 6 à 20 acides aminés.

Si les peptides sélectionnés peuvent ainsi être utilisés tels quels en fonction des critères ci-dessus définis, il peut être toutefois avantageux de modifier lesdits peptides.

Cette modification pourra se faire par substitution. Ainsi, le nombre d'acides aminés étant conservé, un ou plusieurs des acides aminés du peptide sélectionné et de préférence moins du quart des acides aminés et notamment 1, 2 ou 3 acides aminés seront remplacés par un ou plusieurs acides aminés différents. Le ou les acide(s) aminé(s) de substitution comporteront de préférence une fonction libre, telle que NH₂, COOH, SH, par exemple.

Comme acides aminés pouvant substituer un ou plusieurs acides aminés du peptide sélectionné, on peut citer notamment la tyrosine ou les acides aminés permettant un couplage orienté sur une protéine porteuse, une enzyme ou toute autre molécule intéressante.

Il est également intéressant d'ajouter un ou plusieurs acides aminés au peptide sélectionné. Le ou les acide(s) aminé(s) ajoutés seront avantageusement choisis parmi ceux pouvant être utilisés pour les substitutions.

Il peut également être intéressant de modifier ledit fragment par délétion d'un ou plusieurs acides aminés, de préférence moins de la moitié des acides aminés.

Afin d'éviter des interférences lors de l'identification ou du dosage des protéines selon la présente invention, le peptide sélectionné peut être retrouvé dans la séquence d'une protéine mais n'existe pas naturellement à l'état de fragment peptidique.

Comme déjà indiqué, le peptide pourra être sélectionné pour sa spécificité pour une protéine unique. Au contraire, on pourra sélectionner le peptide pour sa spécificité pour une famille de protéines.

En vue de la mise en oeuvre du procédé ci-dessus décrit, le peptide sélectionné pourra se trouver sous forme polymère. Cette forme polymère peut correspondre par exemple à deux fragments de protéines libérables par un traitement approprié associés par un ou plusieurs ponts disulfures par l'intermédiaire de cystéines naturelles.

La polymérisation pourra être également utilisée dans le but de modifier les propriétés immunogéniques d'un ou plusieurs peptides. Le polymère pourra être constitué de monomères identiques ou non. La fixation des monomères pourra se faire, par exemple, grâce aux fonctions amines, carboxylates ou sulfhydryles, et notamment par l'intermédiaire d'une ou plusieurs molécules joignant ces monomères.

Les avantages du procédé ci-dessus décrit sont nombreux.

Il n'y a aucun besoin de détenir physiquement la protéine. Une connaissance bibliographique de la séquence ou d'une partie de la séquence de la ou des protéine(s) à doser suffit. L'homme de l'art, connaissant les techniques de clivage enzymatique grâce à une ou plusieurs enzymes pourra déterminer quels peptides peuvent correspondre à un fragment libérable par traitement approprié.

La sélection du peptide pourra se faire soit parce que la protéine considérée est la seule à posséder ladite séquence, soit au contraire, parce que la séquence est commune à toute une famille de protéines et que l'on veut doser la famille de protéine entière.

Il est également possible de sélectionner a priori une séquence vis-à-vis de critères particuliers tels que le critère de taille du peptide.

Il est possible de modifier le fragment peptidique sélectionné, ce qui permet par exemple de réaliser un couplage à une protéine porteuse de façon à orienter la réponse antigène-anticorps ou de coupler avantageusement une molécule d'intérêt telle qu'une enzyme, ou encore à introduire un acide aminé tel qu'une tyrosine pour effectuer un marquage par l'iode 125.

La technique de la présente invention supprime aussi nombre d'inconvénients des techniques de l'art antérieur tels que l'élimination des protéines gênantes. En effet, dans les techniques de l'art antérieur, il est difficile de trouver une procédure efficace et de longs efforts sont nécessaires pour déterminer la technique de clivage enzymatique et/ou chimique permettant l'élimination des protéines parasites.

La synthèse du peptide sélectionné est de la compétence de l'homme de l'art et peut être réalisée selon les techniques bien connues parmi lesquelles on peut citer notamment la synthèse peptidique en phase solide, selon Merrifield.

Ce procédé est particulièrement adapté à la réalisation de dosages immunologiques de nombreuses hormones protéiques difficiles à purifier et présentant fréquemment des homologies de structure. Un autre cas intéressant est celui du dosage immunologique d'une protéine virale où trois difficultés majeures peuvent se présenter : la première est l'obtention de l'antigène en quantité et en pureté suffisante pour immuniser des animaux, la deuxième est la grande variabilité antigénique que l'on peut rencontrer à travers les différentes souches d'un même virus et la troisième est l'interférence provoquée par les anticorps anti-protéines virales présents dans les sérums des personnes infectées, qui peuvent masquer tout ou partie des épitopes reconnus par les anticorps du dosage. Dans ce cas, également, le dosage d'un fragment protéolytique permet de résoudre ces différents problèmes. En effet, l'utilisation d'un peptide de synthèse correspondant à un fragment protéolytique permet d'avoir un immunogène très pur et en quantité importante ; le choix judicieux de la séquence permet de sélectionner des fragments conservés au sein des différentes souches d'un même virus ou au contraire spécifique d'une souche et les épitopes des anticorps du dosage n'existant pas à l'état naturel, ne peuvent être masqués par les anticorps sériques.

Les exemples qui suivent illustrent la présente invention sans toutefois la limiter.

### Exemple 1 : Dosage de la protéine P25 du virus HIV

### 1. Sélection des fragments peptidiques

Le choix de la technique de fragmentation par la trypsine a été retenu pour son efficacité, son faible coût, sa simplicité et sa spécificité (clivage après les acides aminés arginine et lysine). La carte de digestion trypsique de la protéine P25 de la souche de référence HIV-1 BRU a été établie à partir de la séquence en acides aminés extraite de la banque de données GENPRO, à l'aide du logiciel BISANCE (CITI 2). Seuls les fragments trypsiques ayant une taille supérieure ou égale à six acides aminés ont été sélectionnés. Les fragments retenus ont été ensuite comparés aux séquences en acides aminés des protéines P25 des autres souches de virus HIV-1 et HIV-2 contenues dans les banques de données NBRF et GENPRO. Seuls les fragments trypsiques, ou les peptides de plus de six acides aminés correspondant à une extrémité COOH ou NH₂ d'un fragment trypsique, qui présentaient plus de 80 % d'homologie de séquence à travers les différentes souches de virus HIV-1 et HIV-2 ont été conservés. Ces peptides ont été ensuite comparés à l'ensemble des séquences protéiques contenues dans les banques de données NBRF et GENPRO et aucune homologie significative avec des séquences autres que celles des protéines P25 des virus HIV et des virus SIV n'a été relevée.

Quatre peptides ont été sélectionnés suivant les critères précédents et synthétisés :

### Le peptide T7K

Thr-Leu-Asn-Ala-Trp-Val-Lys
Ce peptide est un fragment trypsique complet correspondant aux acides aminés 19 à 25 de la protéine P25 de la souche de référence HIV-1 BRU. Il est strictement conservé à travers les différentes souches de virus HIV dont les séquences ont été étudiées.

### Le peptide G11C

Gly-Ser-Asp-Ile-Ala-Gly-Thr-Thr-Ser-Thr-Cys
Ce peptide correspond aux acides aminés 101 à 110 de l'extrémité NH2 du fragment trypsique comprenant les acides aminés 101 à 131 pour la souche de référence HIV-1 BRU auxquels a été rajouté en COOH une cystéine supplémentaire permettant des couplages chimiques orientés sur le groupement SH. Ce peptide est également strictement conservé à travers les différentes souches de virus HIV dont les séquences ont été étudiées.

### Le peptide N17K

Asn-Trp-Met-Thr-Glu-Thr-Leu-Leu-Val-Gln-Asn-
Ala-Asn-Pro-Asp-Cys-Lys

Ce peptide est un fragment trypsique complet correspondant aux acides aminés 183 à 199 de la protéine P25 de la souche de référence HIV-1 BRU. Il présente une substitution de l'acide glutamique en position 187 par une glutamine pour le virus HIV-2 ROD ; une substitution de l'acide glutamique en position 187 par une glutamine une substitution de l'asparagine en position 193 par une sérine et une délétion de l'alanine en position 194 par une glutamine pour le virus HIV-2 SBLISY ; une substitution de l'acide glutamique en position 187 par une glutamine et une substitution de la valine en position 191 par une isoleucine pour le virus HIV-2 FG ; et une substitution de l'asparagine en position 183 en glycine pour le virus HIV-1 Z2.

### Le peptide T18K

Thr-Leu-Glu-Glu-Met-Met-Thr-Ala-Cys-Gln-Gly-
Val-Gly-Gly-Pro-Gly-His-Lys

Ce peptide correspond aux acides aminés 210 à 227 de l'extrémité COOH du fragment trypsique composé des acides aminés 204 à 227 pour la souche de référence HIV-1 BRU. Il présente une substitution de la méthionine en position 215 par une leucine et une substitution de l'histidine en position 226 par une glutamine pour le virus HIV-2 ROD; une substitution de la méthionine en position 215 par une leucine et une substitution de la glycine en position 220 par une isoleucine pour le virus HIV-2 SBLISY; une substitution de la méthionine en position 215 par une leucine; une substitution de l'alanine en position 217 par une thréonine et une substitution de l'histidine en position 226 par une glutamine pour le virus HIV-2 FG ; et une substitution de la glycine en position 225 par une sérine pour les virus HIV-1 ELI, HIV-1 MAL et HIV-1 Z2.

### 2. Production d'anticorps

Ces quatre peptides ont été couplés à différentes protéines porteuses afin d'être utilisés comme immunogènes.

En particulier, le peptide N17K a été couplé à de la sérum albumine bovine par l'intermédiaire de succinimidyl-4 (N-maléimido-méthyl) cyclohexane 1-carboxylate (SMCC) qui réagit d'une part avec le groupement SH de la cystéine naturelle du peptide et d'autre part avec les groupements epsilon-NH2 des lysines de la protéine porteuse. A de la sérum albumine bovine (Serva) en solution à 5 mg/ml dans un tampon phosphate 0.1M, pH 7, on ajoute du SMCC (Pierce) en solution à 25 mg/ml dans le DMF dans un rapport molaire de 1/50. Après une incubation d'une heure à température ambiante, les différents réactifs sont séparés par tamisage moléculaire sur une colonne de Biogel® P2 (Bio-Rad®) équilibrée en tampon phosphate 0.1M, pH 6. Le peptide est ensuite ajouté à la solution de BSA-SMCC, dans un rapport molaire de 30/1 et le mélange réactionnel est incubé 12 heures à 4°. La purification de l'immunogène est réalisée par tamisage moléculaire sur une colonne de Biogel® P4 (Bio-Rad®) équilibrée en tampon PBS, pH 7,2. Quatre souris BALB/c ont été immunisées à intervalle mensuel avec 50 µg de cet immunogène injecté en adjuvant complet de Freund, pour moitié par voie sous cutanée et pour moitié par voie intrapéritonéale.

### 3. Caractérisation des anticorps

Les anticorps ainsi obtenus sont caractérisés par leur capacité à lier un traceur radioactif composé du peptide N17K couplé à glycyl-L-tyrosine marqué à l'iode 125. Dans un premier temps, à une solution de glycyl-L-tyrosine (Sigma) 20 mM en tampon phosphate 0.1M à pH 7,0, on ajoute du SMCC (Pierce) en solution à 25 mg/ml dans le DMF dans un rapport molaire de 1/1 et le mélange réactionnel est incubé 2 heures à 20°. Le conjugué glycyl-L-tyrosine SMCC (GT-SMCC) est alors purifié par chromatographie HPLC en phase reverse puis couplé au peptide N17K en solution à 0.5 mM en tampon phosphate 0.1M à pH 6,0 dans un rapport molaire de 1/1. Après 12 heures d'incubation à 4°, le traceur N17K-SMCC-GT est purifié par chromatographie HPLC en phase reverse. Le traceur N17K-SMCC-GT est marqué à l'iode 125 par la méthode à la chloramine T et purifié à nouveau par chromatographie HPLC.

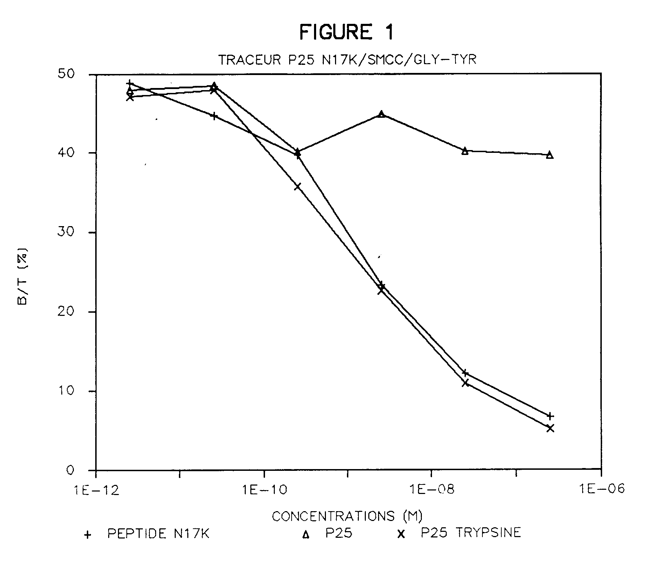

Un exemple de résultat obtenu avec un anti-sérum de souris immunisée avec le conjugué N17K-SMCC-BSA est présenté à la figure 1 : à 100 µl d'anti-sérum de souris dilué au 1/1000 en tampon PBS-BSA 0,1 %, pH 7,2, on ajoute 50 µl de traceur N17K-SMCC-GT radiomarqué, dans le même tampon et 50 µl de peptide N17K ou de P25 HIV-1 BRU (Transgène) ou de P25 après traitement par la trypsine, à différentes concentrations dans du plasma humain normal. Les complexes antigène-anticorps sont précipités après une incubation de 12 heures à 4° par ajout de 200 µl de PEG 6000 à 20 % dans l'eau et centrifugation. La radioactivité du culot est alors mesurée. Comme le montre la figure 1, les anticorps reconnaissent aussi bien le peptide N17K que la protéine P25 traitée par la trypsine mais pas la protéine P25 native.

La détermination quantitative du peptide N17K permet donc de réaliser un dosage de la protéine P25 du virus HIV-1 BRU mais devrait permettre également celui des protéines P25 de toutes les souches de virus HIV qui présentent une homologie de structure avec ce fragment.

### Exemple 2 : Dosage de la LPH (Lipotropine Humaine)

On a développé un dosage immunologique de la LPH en utilisant des anticorps produits contre l'hexapeptide NH2 terminal (E6R) : Glu-Leu-Thr-Gly-Gln-Arg.

Ce peptide qui correspond à un fragment trypsique ne présente aucune homologie significative avec l'ensemble des séquences protéiques contenues dans les banques de données NBRF et GENPRO.

Dans le but d'obtenir des anticorps dirigés contre les extrémités COOH et NH2 de ce fragment, les immunisations ont été pratiquées avec un peptide dont la séquence est la suivante : Glu-Leu-Thr-Cys-Gln-Arg, où une cystéine remplace la glycine présente dans le fragment originel, afin de pouvoir coupler ce peptide à une protéine porteuse par l'intermédiaire du groupement -SH de cette cystéine.

Ce couplage est réalisé par l'intermédiaire de succinimidyl 4 (N-maléimido-méthyl) cyclohexane-1 carboxylate (SMCC) qui réagit, d'une part avec un groupement SH d'une cystéine naturelle ou surnuméraire du peptide et, d'autre part, avec les groupements epsilon NH2 des lysines de la protéine porteuse :

A de la sérum albumine bovine (Serva) (ci-après BSA), en solution à 5 mg/ml dans un tampon phosphate 0,1 M, pH = 7, on ajoute du SMCC (Pierce) en solution à 25 mg/ml dans le DMF dans un rapport molaire de 1/50. Après une incubation d'une heure à température ambiante, les différents réactifs sont séparés par tamisage moléculaire sur une colonne de Biogel® P2 (Bio-Rad®) équilibrée en tampon phosphate 0,1 M, pH = 6. Le peptide est ensuite ajouté à la solution de BSA-SMCC, dans un rapport molaire de 30/1 et le mélange réactionnel est incubé 12 heures à 4°C. La purification de l'immunogène est réalisée par tamisage moléculaire sur une colonne de Biogel® P4 (Bio-Rad®), équilibrée en solution tampon phosphate (PBS), pH = 7,2.

Huit souris BALB/c et deux lapins ont été immunisés à intervalle mensuel avec respectivement 50 et 250 µg de cet immunogène, injecté par voie sous-cutanée en adjuvant complet de Freund.

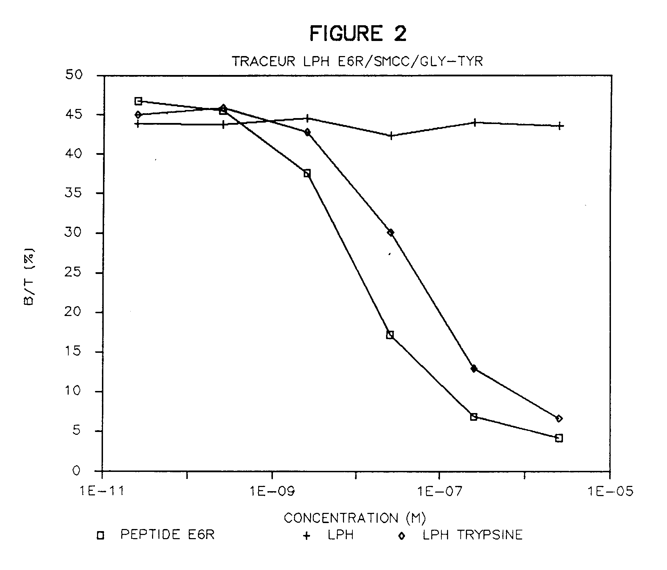

Deux de ces animaux ont produit des anticorps permettant de doser spécifiquement la LPH. Après traitement de l'échantillon sérique par la trypsine qui provoque la libération du peptide NH2 terminal, le dosage est réalisé par compétition avec un peptide identique couplé à glycyl-L-tyrosine par l'intermédiaire de SMCC, radiomarqué à l'iode 125 (figure 2).

On dispose donc, dans cette application de la présente invention, d'un procédé de dosage immunologique de la LPH, pour laquelle il est particulièrement difficile d'obtenir une quantité suffisante de protéine de pureté convenable pour réaliser des immunisations.

## Revendications

1. Procédé d'identification ou de dosage d'une protéine dans lequel:
- on sélectionne dans la protéine, après étude de sa séquence, un peptide libérable par traitement enzymatique protéolytique,
- on prépare par synthèse un peptide de synthèse ayant la séquence du peptide ci-dessus,
- on prépare des anticorps contre ce peptide de synthèse
- on soumet un échantillon contenant éventuellement ladite protéine audit traitement enzymatique pour cliver ladite protéine
- on procède dans ledit échantillon à une détermination immunologique de la présence du peptide libérable, par mise en contact desdits anticorps avec ledit échantillon
- on en déduit si désiré la quantité de protéine présente dans l'échantillon.

2. Procédé selon la revendication 1, caractérisé en ce que le peptide comporte de 6 à 30 acides aminés.

3. Procédé selon la revendication 1 ou 2, caractérisé en ce que le peptide comporte de 6 à 20 acides aminés.

4. Procédé selon l'une quelconque des revendications 1, 2 ou 3, caractérisé en ce que le peptide correspond à une partie de fragment C-terminale, N-terminale ou médiane.

5. Procédé selon l'une quelconque des revendications 1 à 4, caractérisé en ce que le peptide sélectionné est modifié.

6. Procédé selon l'une quelconque des revendications 1 à 5, caractérisé en ce que le peptide est modifié par substitution d'un ou plusieurs acides aminés.

7. Procédé selon l'une quelconque des revendications 1 à 5, caractérisé en ce que le peptide est modifié par adjonction d'un ou plusieurs acides aminés.

8. Procédé selon l'une quelconque des revendications 1 à 5, caractérisé en ce que le peptide est modifié par délétion d'un ou plusieurs acides aminés.

9. Procédé selon l'une quelconque des revendications 1 à 8, caractérisé en ce que le peptide sélectionné peut être retrouvé dans la séquence d'une protéine, mais n'existe pas naturellement à l'état de fragment.

10. Procédé selon l'une quelconque des revendications 1 à 9, caractérisé en ce que le peptide est sélectionné pour sa spécificité pour une protéine unique.

11. Procédé selon l'une quelconque des revendications 1 à 9, caractérisé en ce que le peptide est sélectionné pour sa spécificité pour une famille de protéines.

12. Procédé selon l'une quelconque des revendications 1 à 11, caractérisé en ce que le peptide se trouve sous forme polymère.

13. Procédé selon l'une quelconque des revendications 1 à 12 appliqué à l'identification d'une protéine.

14. Procédé selon l'une quelconque des revendications 1 à 12 appliqué au dosage d'une protéine.

15. Procédé selon l'une quelconque des revendications 1 à 14, caractérisé en ce que la protéine est la protéine P25 d'un virus HIV.

16. Procédé selon l'une quelconque des revendications 1 à 14, caractérisé en ce que la protéine est la Lipotropine Humaine (LPH).

## Claims

1. Process for the identification or determination of a protein in which;
- after study of its sequence, a peptide which can be liberated by proteolytic enzymatic treatment is selected in the protein,
- a synthetic peptide having the sequence of the above peptide is prepared by synthesis,
- antibodies against this synthetic peptide are prepared,
- a sample optionally containing said protein is subjected to said enzymatic treatment in order to cleave said protein,
- an immunological determination of the presence of the releasable peptide is proceeded with in said sample, by putting in contact said antibodies with said sample,
- if desired, the quantity of protein present in the sample is deduced.

2. Process according to claim 1, characterized in that the peptide contains 6 to 30 amino acids

3. Process according to claim 1 or 2, characterized in that the peptide contains 6 to 20 amino acids

4. Process according to any one of claims 1, 2 or 3, characterized in that the peptide corresponds to a part of the C-terminal, N-terminal or median fragment.

5. Process according to any one of claims 1 to 4, characterized in that the selected peptide is modified.

6. Process according to any one of claims 1 to 5, characterized in that the peptide is modified by substitution of one or more amino acids.

7. Process according to any one of claims 1 to 5, characterized in that the peptide is modified by the addition of one or more amino acids.

8. Process according to any one of claims 1 to 5, characterized in that the peptide is modified by the deletion of one or more amino acids.

9. Process according to any one of claims 1 to 8, characterized in that the selected peptide can be found in the sequence of a protein, but does not exist naturally in the state of a fragment.

10. Process according to any one of claims 1 to 9, characterized in that the peptide is selected for its specificity for a single protein.

11. Process according to any one of claims 1 to 9, characterized in that the peptide is selected for its specificity for a family of proteins.

12. Process according to any one of claims 1 to 11, characterized in that the peptide is found in polymer form.

13. Process according to any one of claims 1 to 12 applied to the identification of a protein.

14. Process according to any one of claims 1 to 12 applied to the determination of a protein.

15. Process according to any one of claims 1 to 14, characterized in that the protein is the protein P25 of an HIV virus.

16. Process according to any one of claims 1 to 14, characterized in that the protein is Human Lipotropin (LPH).

## Patentansprüche

1. Verfahren zur Identifizierung oder Quantifizierung eines Proteins, bei dem
- man in dem Protein nach Untersuchung seiner Sequenz ein durch eine enzymatische proteolytische Behandlung freisetzbares Peptid auswählt,
- man durch Synthese ein synthetisches Peptid herstellt, das die Sequenz des obigen Peptids aufweist,
- man Antikörper gegen dieses synthetische Peptid herstellt,
- man eine Probe, die gegebenfalls das genannte Protein enthält, der enzymatischen Behandlung unterzieht, um das Protein zu spalten,
- man in der Probe eine immunologische Bestimmung der Anwesenheit des freisetzbaren Peptids vornimmt, indem man die Antikörper mit der Probe in Kontakt bringt,
- man, falls gewünscht, hieraus die Menge des in der Probe vorhandenen Proteins ableitet.

2. Verfahren nach Anspruch 1, dadurch gekennzeichnet, daß das Peptid 6 bis 30 Aminosäuren aufweist.

3. Verfahren nach Anspruch 1 oder 2, dadurch gekennzeichnet, daß das Peptid 6 bis 20 Aminosäuren aufweist.

4. Verfahren nach einem der Ansprüche 1, 2 oder 3, dadurch gekennzeichnet, daß das Peptid einem Teil eines C-terminalen, N-terminalen oder mittleren Fragments entspricht.

5. Verfahren nach einem der Ansprüche 1 bis 4, dadurch gekennzeichnet, daß das ausgewählte Peptid modifiziert wird.

6. Verfahren nach einem der Ansprüche 1 bis 5, dadurch gekennzeichnet, daß das Peptid durch Substitution einer oder mehrerer Aminosäuren modifiziert wird.

7. Verfahren nach einem Ansprüche 1 bis 5, dadurch gekennzeichnet, daß das Peptid durch Hinzufügung einer oder mehrerer Aminosäuren modifiziert wird.

8. Verfahren nach einem der Ansprüche 1 bis 5, dadurch gekennzeichnet, daß das Peptid durch Deletion einer oder mehrerer Aminosäuren modifiziert wird.

9. Verfahren nach einem der Ansprüche 1 bis 8, dadurch gekennzeichnet, daß das ausgewählte Peptid in der Sequenz eines Proteins wiedergefunden werden kann, jedoch nicht natürlich im Zustand eines Fragments existiert.

10. Verfahren nach einem der Ansprüche 1 bis 9, dadurch gekennzeichnet, daß das Peptid nach seiner Spezifität für ein einzelnes Protein ausgewählt wird.

11. Verfahren nach einem der Ansprüche 1 bis 9, dadurch gekennzeichnet, daß das Peptid nach seiner Spezifität für eine Proteinfamilie ausgewählt wird.

12. Verfahren nach einem der Ansprüche 1 bis 11, dadurch gekennzeichnet, daß das Peptid in polymerer Form vorliegt.

13. Verfahren nach einem der Ansprüche 1 bis 12, verwendet für die Identifizierung eines Proteins.

14. Verfahren nach einem der Ansprüche 1 bis 12, verwendet für die Quantifizierung eines Proteins.

15. Verfahren nach einem der Ansprüche 1 bis 14, dadurch gekennzeichnet, daß das Protein das P25-Protein eines HIV-Virus ist.

16. Verfahren nach einem der Ansprüche 1 bis 14, dadurch gekennzeichnet, daß das Protein das humane Lipotropin (LPH) ist.
